# EUROPEAN PATENT APPLICATION

(11) **EP 3 895 691 A1**
(43) Date of publication of application: **20.10.2021**
(21) Application number: 20169694.5
(22) Date of filing: 15.04.2020
(51) Int. Cl.: A61K 9/00, A61K 31/00, A61K 47/10, A61K 47/32, A61K 47/38

(54) **ULIPRISTAL ACETATE OTF**

(71) Applicant: LTS Lohmann Therapie-Systeme AG, 56626 Andernach (DE)
(72) Inventor: Linn, Michael, 55596 Waldböckelheim (DE); Norelli, Claudia, 56204 Hillscheid (DE); Warnus, Sabine, 56729 Ettringen (DE); Ficker, Mario, 53179 Bonn (DE)
(74) Representative: Held, Stephan

(57) **Abstract**

The invention relates to an oral thin film, comprising a polymer matrix and ulipristal acetate as an active agent, wherein ulipristal acetate is dispersed in the polymer matrix and the polymer matrix is a matrix of water-soluble polymer selected from poly(ethylene oxide), poly(vinyl alcohol) or hydroxypropyl methylcellulose.

The oral thin film is suitable as emergency contraceptive and can be administered in the oral cavity without water. It quickly disintegrates in the oral cavity wherein the active agent ulipristal acetate is mainly swallowed in undissolved form and absorbed into the gastrointestinal tract.

## Description

The invention relates to oral thin films including ulipristal acetate as an active agent.

Oral thin films (OTF) are thin, flexible films based on a polymer matrix and loaded with active substances for drug delivery. The oral thin films are taken orally and dissolve immediately in the mouth or are applied to the mucosa. They are placed on or under the tongue, or buccal where they then dissolve or disintegrate.

EP 422100 B1 discloses the active agent ulipristal acetate. Ulipristal acetate is a well-known emergency contraceptive ("morning-after pill"). It is administered as a tablet ("EllaOne®") containing 30 mg micronized ulipristal acetate and lactose monohydrate, povidone, croscarmellose sodium and magnesium stearate as further ingredients. EllaOne® was approved in the European Union in 2009.

Tablets like EllaOne® are usually taken with water to ease swallowing. In regions where there is no quick access to clean drinking water, taking tablets might thus be challenging, especially for certain patient groups having difficulties with swallowing medications i.e. suffering from dysphagia. Administration of oral thin films, which quickly dissolve upon application in the oral cavity, do not require additional water and is therefore advantageous.

To achieve high patient compliance for administration of an oral thin film, a polymer matrix is necessary, which integrates the active substance (e.g. ulipristal acetate), creates a pleasant sensation in the oral cavity upon administration (pleasant "mouthfeel") and dissolves and/or disintegrates quickly without the addition of drinking water.

US 20150258118 A1 relates to a co-micronization product comprising an active ingredient selected from selective progesterone receptor modulators or metabolites thereof, e.g. ulipristal acetate, and an N-vinyl-2-pyrrolidone-based polymer and a pharmaceutical composition comprising said co-micronization product and an excipient. The examples of dosage forms for the pharmaceutical composition mentioned include inter alia orodispersible films. The method for preparing the co-micronization product includes the steps of mixing the active ingredient with the polymer and co-micronizing the mixture obtained. According to US 20150258118 A1, the co-micronization method significantly improves the in vitro dissolution profile of ulipristal acetate, which should correlate with enhanced in vivo bioavailability.

The object of the present invention was to provide an oral thin film with the active pharmaceutical ingredient ulipristal acetate that can be administered orally without the addition of drinking water and that is bioequivalent to the approved ulipristal acetate tablet ("EllaOne®"), which requires a formulation with minimal transmucosal uptake and a release of ulipristal acetate in the gastrointestinal tract, similar to the already approved tablet EllaOne. In other words, the goal is an oral thin film, which quickly disintegrates in the oral cavity so that the active agent ulipristal acetate is mainly swallowed in undissolved form and absorbed within the gastrointestinal tract.

An oral thin film as defined in claim 1 can achieve this object. Accordingly, the present invention relates to an oral thin film, comprising a polymer matrix and ulipristal acetate as an active agent, wherein ulipristal acetate is dispersed in the polymer matrix and the polymer matrix is a matrix of water-soluble polymer selected from poly(ethylene oxide), poly(vinyl alcohol) or hydroxypropyl methylcellulose.

It has been shown that the water-soluble polymers selected from poly(ethylene oxide), poly(vinyl alcohol), and hydroxypropyl methylcellulose are polymers which in combination with ulipristal acetate form a solid API-matrix combination (API = active pharmaceutical ingredient) that is sufficiently stable for storage and administration. After application in the oral cavity (or in in vitro tests with addition of water, buffer or artificial saliva) the inventive oral thin film disintegrates very quickly and releases the active ingredient ulipristal acetate. The prepared formulations are characterized by the fact that they quickly disintegrate into easily swallowable components such as dissolved polymer and released ulipristal acetate.

Due to the rapid decomposition and the short residence time in the oral cavity, absorption of the active agent by the mucosa is minimized. The active agent ulipristal acetate is mainly swallowed in solid form, preferably crystalline form, and absorbed within the gastrointestinal tract. Thus, bioequivalence to the ulipristal acetate tablet EllaOne® can be accomplished. This could be achieved by using the above mentioned water-soluble polymers which generally also exhibit hygroscopic properties. Thus, the moisture in the oral cavity is sufficient for dissolution.

For the rapidly decomposing OTF, which means rapid dissolution or disintegration in the oral cavity and rapid swallowing, the polymers selected have a good and quick solubility in water and generally hygroscopic properties and therefore absorb sufficient water from the oral cavity to disintegrate. This means that no drinking water is required to ease administration, which is an advantage over a tablet. The oral thin film of the invention is also particularly advantageous for patients, which do not like to take tablets or have difficulties swallowing i.e. suffer from dysphagia.

As indicated, oral thin films in general are known and abbreviated as OTF. Oral thin films that readily dissolve in the oral cavity are also commonly referred as orodispersible films.

The OTF of the invention have e.g. a size in the range of 0.3 to 20 cm², preferably 1 to 10 cm². The thickness of the OTF may be e.g. in the range of 10 to 1000 µm, preferably 40 to 400 µm. The OTF of the invention can take the form of a single-layer or multi-layer film, wherein a single-layer film is preferred.

The OTF of the invention may be a non-foamed or non-porous film. Alternatively, the OTF can comprise a matrix present in the form of a solidified foam having spaces or cavities that are filled with a gas, a gas mixture, a liquid or a liquid mixture. Such OTFs are commonly referred to as "foam-OTFs".

Ulipristal acetate is 17α-acetoxy-11α-(4-N,N-dimethylaminophenyl)-19-norpregna-4,9-dien-3,20-dion) with the following chemical formula:

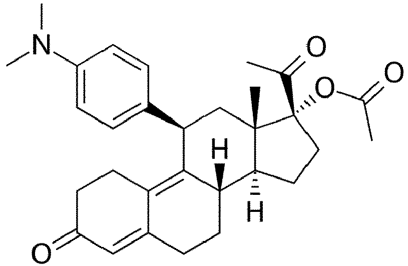

The OTF of the invention comprises a polymer matrix and ulipristal acetate as an active agent. The active agent ulipristal acetate is dispersed in the polymer matrix. In the polymer matrix, ulipristal acetate is present in solid form, in particular in crystalline form, as particles. The particles are firmly attached to the polymer matrix. Optical evaluation shows that the solid or crystalline ulipristal acetate is embedded in the matrix in such a way that a homogeneous OTF is obtained (no visible "API crumbs").

In a preferred embodiment, the ulipristal acetate is micronized ulipristal acetate.

The particle size of the micronized ulipristal acetate, may range e.g. from 0.2 to 100.0 µm, preferably from 0.5 to 40.0 µm. The particle size refers to the volume-weighted particle size d90 and can be determined e.g. by laser diffraction analysis, dynamic light scattering or sieve analysis. A volume-weighted particle size d90 refers to the volume-weighted particle size where 90% of the distribution has a smaller particle size and ten percent has a larger particle size as is known by the skilled person.

The amount of ulipristal acetate is preferably 10 to 60% by weight, more preferably 20 to 43% by weight, based on the total weight of the oral thin film. In the case where the water-soluble polymer is selected from poly(ethylene oxide) or poly(vinyl alcohol), the amount of ulipristal acetate is preferably 10 to 60% by weight, more preferably 20 to 43% by weight, still more preferably 25 to 35% by weight, based on the total weight of the oral thin film. In the case where the water-soluble polymer is selected from hydroxypropyl methylcellulose, the amount of ulipristal acetate is preferably 20 to 60% by weight, more preferably 20 to 43% by weight or 35 to 43% by weight, based on the total weight of the oral thin film.

The polymer matrix in which ulipristal acetate is dispersed is a matrix of water-soluble polymer selected from poly(ethylene oxide), poly(vinyl alcohol) or hydroxypropyl methylcellulose. These polymers are film forming polymers. Moreover, these polymers are generally hygroscopic.

Herein, a water-soluble polymer refers to a polymer having a solubility in water of at least 10 g/L, preferably at least 50 g/L, at a temperature of 25 °C.

It is preferred that the water-soluble polymer is selected from poly(ethylene oxide), poly(vinyl alcohol) or hydroxypropyl methylcellulose. One, two or more types of poly(ethylene oxide), one, two or more types of poly(vinyl alcohol) or one, two or more types of hydroxypropyl methylcellulose may be used.

The amount of water-soluble polymer is preferably 20 to 90% by weight, more preferably 40 to 70% by weight, based on the total weight of the oral thin film. In the case where the water-soluble polymer is selected from poly(ethylene oxide) and/or poly(vinyl alcohol), the amount of water-soluble polymer is preferably 25 to 90% by weight, more preferably 35 to 70% by weight, based on the total weight of the oral thin film. In the case where the water-soluble polymer is selected from hydroxypropyl methylcellulose, the amount of water-soluble polymer is preferably 30 to 70% by weight, more preferably 40 to 60% by weight, based on the total weight of the oral thin film.

The poly(ethylene oxide) (PEO) used as water-soluble polymer preferably has a molecular weight in the range of 50,000 to 200,000 Dalton, preferably 75,000 to 150,000 Dalton. A particular preferred poly(ethylene oxide) is poly(ethylene oxide) WSR N-10 (PEO WSR N-10), commercially available as Polyox® WSR N-10 or Polyox® WSR N-10 NF, respectively from Dow Chemical Company, for which a molecular weight of about 100,000 Dalton is given.

The poly(vinyl alcohol) (PVA) used as water-soluble polymer preferably has a molecular weight in the range of 20,000 to 40,000 Dalton, preferably 25,000 to 35,000 Dalton, and/or a degree of hydrolysis of 84 to 92 mol-%, preferably 86 to 90 mol-%. A particular preferred poly(vinyl alcohol) is poly(vinyl alcohol) 4-88 (PVA 4-88), commercially available e.g. as Mowiol® 4-88 from Merck KGaA, for which a molecular weight of about 31,000 Dalton and a degree of hydrolysis of about 86.7-88.7 mol % is given.

The hydroxypropyl methylcellulose (HPMC) used as water-soluble polymer preferably has a labelled viscosity in the range of 1 to 100 mPas, preferably 2 to 75 mPas. The labelled viscosity refers to the viscosity as determined according to USP monograph <911> method 1, of 2012 (USP = US pharmacopeia). The hydroxypropyl methylcellulose (HPMC) preferably is of substitution type 2910 according to USP: substitution degree: methoxy 28-30%, hydroxypropoxy 7-12%.

Particular preferred hydroxypropyl methylcelluloses are hydroxypropyl methylcellulose 603 (HPMC 603) (labelled viscosity of 3 mPas and substitution type 2910) and hydroxypropyl methylcellulose 60SH50 (HPMC 60SH50) (labelled viscosity of 50 mPas and substitution type 2910), commercially available e.g. as Pharmacoat® 603 or Pharmacoat® 603W and Metolose® 60SH-50 from Shin-Etsu Chemical Co., Ltd, respectively.

In a preferred embodiment a mixture of two hydroxypropyl methylcelluloses are used as water-soluble polymer, preferably a mixture of HPMC 603 and HPMC 60SH50, e.g. in a weight ratio of HPMC 603 to HPMC 60SH50 of 3/1 to 1/1, preferably 2/1 to 1.5/1.

In a preferred embodiment, the oral thin layer further comprises one or more plasticizers. The plasticizer is also suitable for a reduction of melting temperature and glass transition temperature. Examples for suitable plasticizers are triacetin, triethyl citrate, acetyltributylcitrate, water, ethanol, polyalcohols such as glycerol, diethylene glycol, polyethylene glycol, propylene glycol or glycerol monoesters with fatty acids, wherein glycerol is preferred.

If present, the total amount of plasticizers, preferably glycerol, usually ranges between 0.5 and 20% by weight, preferably 3 to 15% by weight, based on the total weight of the oral thin film. In the case where the water-soluble polymer is selected from poly(ethylene oxide) and/or poly(vinyl alcohol), the total amount of plasticizer, preferably glycerol, if present, is preferably 0.5 to 15% by weight, more preferably 3 to 11% by weight, based on the total weight of the oral thin film. In the case where the water-soluble polymer is selected from hydroxypropyl methylcellulose, the total amount of plasticizer, preferably glycerol, if present, is preferably 3 to 15% by weight, more preferably 4 to 13% by weight, based on the total weight of the oral thin film.

The oral thin layer may further comprise one or more further excipients, which are common is this technical field. Example for suitable excipients are taste-masking agents, sweetening agents, flavoring agents, lubricants, pigments, coloring agents, stabilizers, fillers, saliva stimulating agents, emulsifiers, surfactants, enhancers, pH regulating agents, buffers, buffering agents, release modifiers, softeners, moisturizers, mold release agents, adhesives, anti-adherents and antioxidants. Preferably, one or more sweetening agents are used in the oral thin film. The total amount of optional further excipients, such as sweetening agents, may be not more 15% by weight, preferably not more than 5% by weight, based on the total weight of the oral thin film.

Example for antioxidants are sodium metabisulfite, butylated hydroxytoluene (BHT), butylated hydroxyanisole (BHA), ascorbic acid, tocopherols.

A benefit of the inventive oral thin film is its storage stability. The main degradation product of ulipristal acetate is N-demethyl ulipristal acetate (DMUA) having the following formula

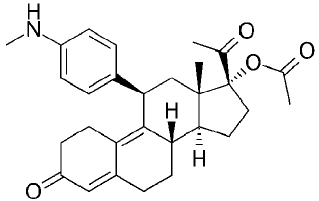

In a preferred embodiment, the oral thin film is characterized in that after storing of the oral thin film at a temperature of 40 °C and 75% relative humidity for 6 months the amount of the degradation product N-demethyl ulipristal acetate (DMUA) is less than 1% by weight based on the initial amount of ulipristal acetate in the oral thin film before storage.

The invention also relates to an oral thin film, comprising a polymer matrix and ulipristal acetate as an active agent, wherein ulipristal acetate is dispersed in the polymer matrix, and wherein after storing of the oral thin film at a temperature of 40 °C and 75% relative humidity for 6 months the amount of the degradation product N-demethyl ulipristal acetate (DMUA) is less than 1% by weight based on the initial amount of ulipristal acetate in the oral thin film before storage.

The invention also relates to method for preparing an oral thin film according to the invention as described above, comprising the following steps:
a) mixing water-soluble polymer, solvent comprising or consisting of water or a mixture of water and one or more organic solvents, and solid ulipristal acetate, preferably micronized ulipristal acetate, to obtain a suspension, wherein water-soluble polymer is dissolved in the solvent and ulipristal acetate is suspended in the solvent,
b) casting or coating the suspension obtained on a support, a coating liner, or in a mold to spread the suspension, and
c) evaporating the solvent.

The water-soluble polymer is usually selected from poly(ethylene oxide), poly(vinyl alcohol) or hydroxypropyl methylcellulose.

The solvent used comprises or consists of water or a mixture of water and one or more organic solvents, wherein use of water is preferred. Examples for a suitable organic solvent are alcohols, in particular ethanol. The weight ratio of water to organic solvent, preferably ethanol, if used, may be e.g. in the range of 95/5 to 5/95, preferably in the range of 95/5 to 80/20.

In step a), the ingredients water-soluble polymer, solvent, and solid ulipristal acetate are mixed with each other to obtain a suspension. The order for combining the ingredients is arbitrary. For instance, at first the water-soluble polymer may be dissolved in the solvent and then solid ulipristal acetate is added, or alternatively, at first solid ulipristal acetate is provided and then solvent and polymer are added.

One or more plasticizers, preferably glycerol, are preferably incorporated in the suspension. The addition may be effected at any time before step b). Optionally, one or more further excipients such as sweetening agents may be added at any order.

In case of preparing a foam OTF (foamed film), the suspension is generally foamed with a gas. Foaming is generally carried out before pouring step b). Examples for a suitable gas for foaming are air, N₂, Argon, or CO₂.

After evaporation of solvent a solid API-polymer matrix combination is achieved, where ulipristal acetate particulates are dispersed in the polymer matrix. A firm attachment of ulipristal acetate particulates in the matrix is achieved. The films obtained can be cut and/or punched into pieces of desired dimensions.

Drying conditions for solvent evaporation can be e.g. in the range of 40 to 100 °C, more preferably 50 to 80 °C, most preferably 50 to 75°C. It is also possible that drying is effected by applying a temperature gradient.

After solvent evaporation, the residual solvent content in the oral thin film obtained is preferably in the range of 0.2 to 10% by weight, preferably 0.8 to 6 % by weight, based on the total weight of the oral thin film. The residual solvent contained may be water or water and one or more organic solvents such as water and ethanol.

The invention also relates to an oral thin film according to the invention as described above for use as an emergency contraceptive, i.e. for use in the prevention of pregnancy after sex, in particular after unprotected sex. The oral film will be administered in the oral cavity. Addition of drinking water is not necessary. The active agent ulipristal acetate is mostly swallowed into the gastrointestinal tract in solid form so that bioequivalence to the approved ulipristal acetate tablet ("EllaOne®") is accomplished.

The invention will now be explained more specifically with reference to the following examples, which are given for illustration of this invention and are not intended to be limiting thereof.

### Examples

In all examples ulipristal acetate having the following particle size distribution as measured by laser diffraction was used:
d₁₀ = 1.48 µm ± 11.20%; d₅₀ = 3.64 µm ± 8.71%; d₉₀ = 6.73 µm ± 10.40%; d₉₅ = 7.86 µm ± 11.08%; d₉₉ = 10.52 µm ± 16.30%.

OTF laminates were prepared by standard laboratory methods (stirrers, glass vessels, coating tools, drying oven). The formulations were prepared as suspension formulations by mixing API, matrix polymer and excipients in a process solvent for a suitable time, then coating the prepared mass on a suitable liner, followed by drying in a drying oven. This process yielded laminate pieces that were punched into OTF of a suitable size (about 7 cm²). PVA 4-88 and Polyox WSR N10 was used as pre-solution (PVA 4-88: 35 % in water, Polyox WSR N10: 21 % in water). For foam formulations, the suspension obtained in step b) was foamed by a whisk or turbine stirrer with air or nitrogen gas.

### Example 1 - PEO-based OTF

An oral thin film having the following formulation (calculated as dry basis) was prepared as suspension formulation with water as process solvent (solid content 30.4 %), alternatively ethanol/water 10/90 is suitable as process solvent (solid content 33%):

| **ingredient** | **function** | **proportion [% by weight]** |
|---|---|---|
| Polyox® WSR N-10 | matrix polymer | 62.0 |
| ulipristal acetate (micronized) | API | 30.0 |
| glycerol | plasticizer (reduces melting temperature and glass transition temperature) | 5.0 |
| saccharin Na | sweetening agent | 2.0 |
| sucralose | sweetening agent | 1.0 |

It was found that PEO WSR N-10 (Polyox®WSR N10) is a suitable matrix polymer for ulipristal acetate OTF formulations. An ulipristal acetate content of 30% was found to yield a haptically and optically good OTF film.

### Example 2 - PVA-based OTF foam

An oral thin film having the following formulation (calculated as dry composition) was prepared as suspension formulation with water as process solvent (solid content 40 %), air was used for foaming:

| **ingredient** | **function** | **proportion [% by weight]** |
|---|---|---|
| PVA 4-88 | matrix polymer | 63.0 |
| ulipristal acetate (micronized) | API | 30.0 |
| glycerol | plasticizer (reduces melting temperature and glass transition temperature) | 4.0 |
| saccharin Na | sweetening agent | 2.0 |
| sucralose | sweetening agent | 1.0 |

PVA 4-88 was found a suitable polymer for formulating an ulipristal acetate OTF as a foam. An API loading with 30% ulipristal acetate was found suitable. The PVA formulation as OTF foam with 30% ulipristal acetate loading had good optical and haptical properties.

Tests with higher loading of 40% ulipristal acetate loading leads to tear resistant, but slightly brittle OTF. Formulations using 30% API as above result in better OTFs.

Furthermore, formulations with 23.7 % API loading and nitrogen gas as foaming gas were found suitable and gave tear resistant films:
Formulation with PVA 4-88 as Matrix polymer and 23.7% API loading with water as process solvent (solid content 40 %), nitrogen was used for foaming:

| **ingredient** | **function** | **proportion [% by weight]** |
|---|---|---|
| PVA 4-88 | matrix polymer | 64.2 |
| ulipristal acetate (micronized) | API | 23.7 |
| glycerol | plasticizer (reduces melting temperature and glass transition temperature) | 8.8 |
| saccharin Na | sweetening agent | 2.2 |
| sucralose | sweetening agent | 1.1 |

### Example 3 - PVA-based OTF (not foamed)

An oral thin film having the following formulation (calculated as dry composition) was prepared as suspension formulation with water as process solvent (solid content 40 %):

| **ingredient** | **function** | **proportion [% by weight]** |
|---|---|---|
| PVA 4-88 | matrix polymer | 63.0 |
| ulipristal acetate (micronized) | API | 30.0 |
| glycerol | plasticizer (reduces melting temperature and glass transition temperature) | 4.0 |
| saccharin Na | sweetening agent | 2.0 |
| sucralose | sweetening agent | 1.0 |

PVA 4-88 was found a suitable polymer for formulating an ulipristal acetate OTF. An API loading with 30% ulipristal acetate was found suitable. PVA formulations as film with 30% ulipristal acetate loading had good optical and haptical properties.

Tests with higher loading of 40% ulipristal acetate leads to tear resistant, but slightly brittle OTF. Formulations using 30% API result in better OTFs.

### Example 4 - HPMC-based OTF

An oral thin film having the following formulation (calculated as dry composition) was prepared as suspension formulation with water as process solvent (solid content 31.9 %), alternatively ethanol/water 20/80 is suitable as process solvent (solid content 32%):

| **Ingredient** | **Function** | **Proportion [% by weight]** |
|---|---|---|
| HPMC 603 | matrix polymer | 30.55 |
| HPMC 60SH50 | matrix polymer | 16.45 |
| ulipristal acetate (micronized) | API | 40.0 |
| glycerol | plasticizer(reduces melting temperature and glass transition temperature) | 10.0 |
| saccharin sodium | sweetening agent | 2.0 |
| sucralose | sweetening agent | 1.0 |

HPMC was found a suitable polymer for formulating an ulipristal acetate OTF. It was found that an API loading of 40% gives a stable and haptical good film. The glycerol content was set to 10% in order to improve haptic properties and tear-resistance. A process solvent mixture of 20% ethanol and 80% water (solid content 32 %) was found most suitable for manufacturing.

Tests with loading of 30% and 40% ulipristal acetate leads to acceptable films, but formulations using 26.63 % API results in improved OTF:
An oral thin film having the following formulation (calculated as dry composition) was prepared as suspension formulation with water as process solvent (solid content 32 %):

| **Ingredient** | **Function** | **Proportion [% by weight]** |
|---|---|---|
| HPMC 603 | matrix polymer | 37.17 |
| HPMC 60SH50 | matrix polymer | 20.73 |
| ulipristal acetate (micronized) | API | 26.63 |
| glycerol | plasticizer(reduces melting temperature and glass transition temperature) | 12.23 |
| saccharin sodium | sweetening agent | 2.16 |
| sucralose | sweetening agent | 1.08 |

### Comparative example - Kollicoat®IR-based formulations

Kollicoat®IR from BASF, which is a water-soluble polyvinyl alcohol/polyethylene glycol copolymer was tested for preparing an ulipristal acetate OTF. Different ulipristal acetate loadings were investigated, such as 50%, 30% and 20% ulipristal acetate in the formulations. However, these formulations were brittle and without sufficient tear resistance. As a result, Kollicoat®IR was not found a suitable polymer for formulating an ulipristal acetate OTF.

### Example 5 - Permeation study

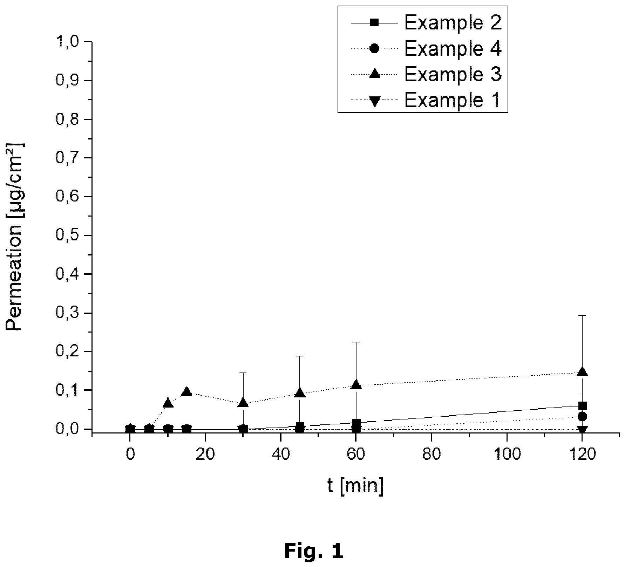

The permeated amount of the OTFs prepared according Examples 1, 2, 3 and 4 were determined by in vitro experiments in accordance with the OECD Guideline (adopted April 13, 2004) using pig mucosa (mucosa oesophagus). A dermatome was used to prepare mucosa to a thickness of 400 µm, with an intact barrier function for all transmucosal therapeutic systems. Die cuts with an area of 0.524 cm² were punched from the OTFs, applied to the mucosa and the mucosa with the OTF was immersed on the top side in artificial saliva (the bottom side being in contact with receptor medium, and the top side being compartmentalized to a mucosa area of 0.985 cm²). The permeated amount of Ulipristal Acetate in the receptor medium (phosphate buffer solution pH 7.4) at a temperature of 37 ± 1°C was measured. The results are shown in Figure 1.

The in vitro experiments show that the four tested formulations showed a very slow, almost non-detectable, permeation of ulipristal acetate (the high standard deviation of the measurement is due to the low amounts of permeated ulipristal acetate, which were in the range of the detection limit). In the first 5 minutes, no detectable amounts of ulipristal acetate were found for all four formulations. Even after 120 minutes only trace amounts (<0.15 pg/_{CM}²) were found. As disintegration of the films is fast (example 6) it can be concluded that the ulipristal acetate is swallowed before relevant amounts can permeate and, thus, PEO, PVA and HPMC are suitable matrix polymers for an immediate release product where the ulipristal acetate is swallowed and absorbed through the gastrointestinal tract.

### Example 6 - Disintegration study

Disintegration times of the OTFs prepared according to Examples 1, 2, 3 and 4 were measured, based on USP 701, using a tablet disintegration instrument (Pharma-Test DIST-3 Triple Basket Tablet Disintegration Tester, 30 strokes per min over a distance of 55 mm, in 11 ELGA Water (RWSx002)). Die cuts of the OTFs with a size of 7.04 cm² were placed in a basket ("sinker") and positioned in a glass tube affixed to the instrument. Finally, time was taken until there were only residues of the OTF inside the basket.

The times indicated in the following table refer to the point when there were only residues of the OTF inside the basket:

| OTF | time to complete disintegration |
|---|---|
| Example 1 (PEO-based) | 76 s |
| Example 2 (PVA-based foam) | 125 s |
| Example 3 (PVA-based film) | 53 s |
| Example 4 (HPMC-based) | 54 s |

These studies show that the polymers tested are fast dissolving and suitable for releasing the API upon fast disintegration in the mouth.

### Example 7 - Stability test

Stability of the OTFs prepared according to Examples 1, 2 and 4 was measured by storing samples of the OTFs at a temperature of 40 °C and 75% relative humidity. At fixed time intervals of 1, 2, 3 and 6 months, samples of the OTFs were analyzed by HPLC for degradation products. The main degradation product detected was N-demethyl ulipristal acetate (DMUA). Tables 1, 2 and 3 show the amounts of DMUA and the total amounts of degradation product detected (Sum) in % by weight based on the initial amount of ulipristal acetate in the OTFs before storage. The tested formulations were found to be stable, with only minor amounts of degradation products.

**Table 1: Summarized values from stability testing protocol for example 2 (PVA formulation) at 40 °C / 75% r.h.**

| **Test Parameter** | **Inital** | **1 Month** | **2 Months** | **3 Months** | **6 Months** |
|---|---|---|---|---|---|
| Related substances | **Sum: 0.21** | **Sum: 0.34** | **Sum: 0.64** | **Sum: 0.90** | **Sum: 1.17** |
| N-Demethyl-Ulipristalacetat | 0.16 | 0.23 | 0.32 | 0.37 | 0.48 |
| Deprotector* | 0.02 | 0.02 | 0.02 | 0.01 | 0.02 |

| | | | | | |
|---|---|---|---|---|---|
| *Acetyl group removed ("deprotected") | | | | | |

**Table 2: Summarized values from stability testing protocol for example 1 (PEO formulation) at 40 °C / 75% r.h.**

| **Test Parameter** | **Inital** | **1 Month** | **2 Months** | **3 Months** | **6 Months** |
|---|---|---|---|---|---|
| Related substances | **Sum: 0.22** | **Sum: 0.26** | **Sum: 0.36** | **Sum: 0.43** | **Sum: 0.47** |
| N-Demethyl-Ulipristalacetat | 0.16 | 0.19 | 0.20 | 0.22 | 0.23 |
| Deprotector* | 0.02 | 0.02 | 0.02 | 0.02 | 0.03 |

| | | | | | |
|---|---|---|---|---|---|
| *Acetyl group removed ("deprotected") | | | | | |

**Table 3: Summarized values from stability testing protocol for example 4 (HPMC formulation) at 40 °C / 75% r.h.**

| **Test Parameter** | **Inital** | **1 Month** | **2 Months** | **3 Months** | **6 Months** |
|---|---|---|---|---|---|
| Related substances | **Sum: 0.18** | **Sum: 0.23** | **Sum: 0.34** | **Sum: 0.37** | **Sum: 0.40** |
| N-Demethyl-Ulipristalacetat | 0.13 | 0.15 | 0.17 | 0.18 | 0.19 |
| Deprotector* | 0.01 | 0.02 | 0.03 | 0.03 | 0.03 |

| | | | | | |
|---|---|---|---|---|---|
| *Acetyl group removed ("deprotected") | | | | | |

## Claims

1. An oral thin film, comprising a polymer matrix and ulipristal acetate as an active agent, wherein ulipristal acetate is dispersed in the polymer matrix and the polymer matrix is a matrix of water-soluble polymer selected from poly(ethylene oxide), poly(vinyl alcohol) or hydroxypropyl methylcellulose.

2. The oral thin film according to claim 1, wherein ulipristal acetate is micronized ulipristal acetate.

3. The oral thin film according to any preceding claim, wherein the amount of ulipristal acetate is 10 to 60% by weight, preferably 20 to 43% by weight, based on the total weight of the oral thin film.

4. The oral thin film according to any preceding claim, wherein in the case where the water-soluble polymer is selected from poly(ethylene oxide) or poly(vinyl alcohol), the amount of ulipristal acetate is 10 to 60% by weight, preferably 20 to 43% by weight, based on the total weight of the oral thin film, and/or in the case where the water-soluble polymer is selected from hydroxypropyl methylcellulose, the amount of ulipristal acetate is 20 to 60% by weight, preferably 20 to 43% by weight, based on the total weight of the oral thin film.

5. The oral thin film according to any preceding claim, wherein the amount of water-soluble polymer is preferably 20 to 90% by weight, more preferably 40 to 70% by weight, based on the total weight of the oral thin film.

6. The oral thin film according to any preceding claim, wherein
in the case where the water-soluble polymer is selected from poly(ethylene oxide) and/or poly(vinyl alcohol), the amount of water-soluble polymer is preferably 25 to 90% by weight, more preferably 35 to 70% by weight, based on the total weight of the oral thin film, and/or
in the case where the water-soluble polymer is selected from hydroxypropyl methylcellulose, the amount of water-soluble polymer is preferably 30 to 70% by weight, more preferably 40 to 60% by weight, based on the total weight of the oral thin film.

7. The oral thin film according to any preceding claim, wherein
the poly(ethylene oxide) (PEO) has a molecular weight in the range of 50,000 to 200,000 Dalton, preferably 75,000 to 150,000 Dalton,
the poly(vinyl alcohol) (PVA) has a molecular weight in the range of 20,000 to 40,000 Dalton, preferably 25,000 to 35,000 Dalton, and/or a degree of hydrolysis of 84 to 92 mol-%, preferably 86 to 90 mol-%, and/or the hydroxypropyl methylcellulose (HPMC) has a labelled viscosity in the range of 1 to 100 mPas, preferably 2 to 75 mPas.

8. The oral thin film according to any preceding claim, wherein the water-soluble polymer is selected from poly(ethylene oxide) WSR N-10, poly(vinyl alcohol) 4-88, hydroxypropyl methylcellulose 603, hydroxypropyl methylcellulose 60SH50 or a mixture of hydroxypropyl methylcellulose 603 and hydroxypropyl methylcellulose 60SH50.

9. The oral thin film according to any preceding claim, further comprising one or more plasticizers, preferably glycerol.

10. The oral thin film according to any preceding claim, wherein the oral thin film is a non-foamed film or a foamed film.

11. The oral thin film according to any preceding claim, wherein after storing of the oral thin film at a temperature of 40 °C and 75% relative humidity for 6 months the amount of the degradation product N-demethyl ulipristal acetate (DMUA) is less than 1% by weight based on the initial amount of ulipristal acetate in the oral thin film before storage.

12. An oral thin film, comprising a polymer matrix and ulipristal acetate as an active agent, wherein ulipristal acetate is dispersed in the polymer matrix, and wherein after storing of the oral thin film at a temperature of 40 °C and 75% relative humidity for 6 months the amount of the degradation product N-demethyl ulipristal acetate (DMUA) is less than 1% by weight based on the initial amount of ulipristal acetate in the oral thin film before storage.

13. A method for preparing an oral thin film according to one of claims 1 to 12, comprising the following steps:
a) mixing water-soluble polymer, solvent comprising or consisting of water or a mixture of water and one or more organic solvents, and solid ulipristal acetate, preferably micronized ulipristal acetate, to obtain a suspension, wherein water-soluble polymer is dissolved in the solvent and ulipristal acetate is suspended in the solvent,
b) casting or coating the suspension obtained on a support, coating liner or in a mold to spread the suspension, and
c) evaporating the solvent.

14. The method of claim 13, where one or more plasticizers are added in mixing step a).

15. The method of claim 13 or claim 14, wherein the suspension is foamed with a gas before step b).

16. An oral thin film according to one of claims 1 to 12 for use as an emergency contraceptive.
